# FASCICULE DE BREVET EUROPEEN

(11) **EP 0 789 008 B1**
(45) Date de publication et mention de la délivrance du brevet: **06.09.2000**
(21) Numéro de dépôt: 97400253.7
(22) Date de dépôt: 05.02.1997
(51) Int. Cl.: C07C 6/12

(54) **Procédé de dismutation et/ou transalkylation d'hydrocarbures alkylaromatiques mettant en oeuvre un catalyseur à base de zéolithe mazzite renfermant au moins un métal des groupes IIA, IVB, IIB ou IVA**
Verfahren zur Dismutation und/oder Transalkylierung von alkylaromatischen Kohlenwasserstoffen mit Verwendung eines auf Zeolit Mazzit basierten Katalysators der mindestens ein Metall der Gruppen IIA, IVB, IIB, oder IVA enthält
Process for the dismutation and/or transalkylation of alkylaromatic hydrocarbons using a catalyst based on a mazzite zeolite comprising at least one metal of the groups IIa, IVB, IIB or IVA

(30) Priorité: 09.02.1996 FR 9601605
(43) Date de publication de la demande: 13.08.1997
(73) Titulaire: INSTITUT FRANCAIS DU PETROLE, 92502 Rueil-Malmaison (FR)
(72) Inventeur: Benazzi, Eric, 78360 Montesson (FR); Alario, Fabio, 92200 Neuilly sur Seine (FR)

(56) Documents cités:
- DE-A- 2 558 035
- FR-A- 2 291 957
- FR-A- 2 303 782
- US-A- 5 210 356

## Description

L'invention concerne l'utilisation en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation du toluène et de triméthylbenzènes pour produire des xylènes, d'un catalyseur comprenant au moins une zéolithe de type structural mazzite, sous forme acide, dont on a modifié les propriétés catalytiques par dipôt, sur la surface extérieure de ses cristaux, d'au moins un métal choisi parmi les métaux du groupe lla, tel que Be, Mg, Ca, Sr ou Ba, du groupe lVb, tel que Ti, Zr ou Hf, du groupe llb tel que Zn, Cd ou Hg et du groupe IVa tel que Ge, Sn ou Pb de la classification périodique des éléments, ledit catalyseur comprenant aussi au moins une matrice et éventuellement au moins un élément pris dans l'ensemble formé par les groupes lB et VIII de la classification périodique des éléments.

Le catalyseur utilisé selon l'invention a été décrit partiellement dans la demande de brevet européen de la demanderesse EP-A-0.569.268, et dans le brevet US-A-5.391.528. Ledit catalyseur comporte au moins une zéolithe oméga sous forme acide de type structural mazzite, dont le diamètre des pores principaux est d'environ 7,4 Å et qui posséde un réseau microporeux monodimensionnel ("Atlas of Zeolites Structure Types", W.M. Meier et D.H. Olson, 3ème Edition, 1992). La zéolithe oméga entrant dans la composition du catalyseur possède un rapport Si/Al atomique, global, compris entre 3,2 et 100 et de préférence entre 6 et 80 et de manière encore plus préférée entre 8 et 60. La teneur en sodium de ladite zéolithe est généralement inférieure à 0,6 % poids et de préférence inférieure à 0,1% poids. Le catalyseur comprend aussi une matrice et éventuellement au moins un élément pris dans l'ensemble formé par les groupes lB et VIII de la classification périodique des élèments. Le catalyseur tel que décrit dans les documents cités précédemment est utilisé en isomérisation d'une coupe C8 aromatique.

De façon surprenante, il est possible, grâce aux dépôts sur la surface extérieure des cristaux de la zéolithe de type structural mazzite, de préférence de la zéolithe oméga, d'au moins un métal choisi parmi les métaux du groupe IIa, IVb, IIb et IVa, et en particulier, de Ge, Zr et/ou Sn, d'obtenir des catalyseurs actifs et sélectifs pour la dismutation d'hydrocarbures alkylaromatiques ou la transalkylation d'hydrocarbures alkylaromatiques.

La présente invention concerne donc l'utilisation en dismutation et/ou de transalkylation d'hydrocarbures alkylaromatiques d'un catalyseur comportant au moins une zéolithe de type structural mazzite, de préférence une zéolithe oméga, au moins en partie, de préférence pratiquement totalement, sous forme acide, telle qu'elle comprend, sur la surface extérieure de ses cristaux, au moins un élément choisi parmi les métaux des groupes IIa, IVb, IIb et IVa de la classification périodique des éléments, ledit catalyseur comprenant aussi au moins une matrice et éventuellement au moins un élément choisi dans l'ensemble formé par les groupes lB et VIII de la classification périodique des éléments.

La dismutation et/ou transalkylation d'hydrocarbures alkylaromatiques est opérée généralement selon les conditions suivantes : une température comprise entre 250 et 600°C, de préférence entre 330 et 500°C ; une pression comprise entre 10 et 60 bar, de préférence entre 20 et 45 bar ; une vitesse spatiale d'alimentation, exprimée en kilogramme de charge introduite par kilogramme de catalyseur et par heure, comprise entre 0,1 et 10 et de préférence entre 0,5 et 4 un rapport molaire hydrogène sur hydrocarbures compris entre 2 et 20 et de préférence entre 3 et 12.

Le catalyseur utilisé selon l'invention, à base de zéolithe de type structural mazzite, de préférence à base de zéolithe oméga, présente des performances catalytiques améliorées, en particulier des sélectivités améliorées, par rapport aux catalyseurs à base de zéolithe oméga de l'art antérieur.

En particulier, le catalyseur utilisé selon la présente invention se révèle très efficace pour la dismutation du toluène et/ou la transalkylation du toluène et des hydrocarbures alkylaromatiques en C9+ (c'est à dire à au moins 9 atomes de carbone par molécule)., en particulier du toluène et de triméthylbenzènes. Ceci se traduit par une diminution importante des réactions secondaires indésirables telles que la formation de polyalkylaromatiques contenant au moins 9 atomes de carbone par molécule.

Le catalyseur utilisé selon la présente invention a déjà été décrit dans la demande de brevet européen de la demanderesse EP-A-0.569.268, et dans le brevet US-A-5.391.528, dans le cas particulier, préféré selon la présente invention, où la zéolithe de type structural mazzite est la zéolithe oméga. Néanmoins, à titre indicatif, ses caractéristiques sont brièvement reprises ci-après. Ledit catalyseur contient généralement de 10 à 99% de zéolithe de stucture mazzite et de préférence encore de 20 à 95%. Dans le cas où le catalyseur contient au moins un élément choisi dans l'ensemble formé par les groupes IB et VIII de la classification périodique des éléments, la teneur pondérale du(des)dit(s) élément(s) est généralement comprise entre 0,01% et 10%, de préférence entre 0,05% et 7% et de préférence encore entre 0,10% et 5%. Le complément à 100 % poids consiste généralement en la part de matrice dans le catalyseur.

La matrice comprise dans le catalyseur utilisé selon l'invention est choisie généralement parmi les éléments du groupe formé par les argiles (par exemple parmi les argiles naturelles telles que le kaolin ou la bentonite), la magnésie, les alumines, les silices, l'oxyde de titane, l'oxyde de bore, la zircone, les phosphates d'aluminium, les phosphates de titane, les phosphates de zirconium et les silice-alumines, de préférence parmi les éléments du groupe formé par les alumines et les argiles.

La zéolithe de type structural mazzite comprise dans le catalyseur utilisé selon l'invention est généralement choisie dans le groupe formé par la zéolithe oméga, la mazzite, la zéolithe LZ-202, la zéolithe mazzite gallosilicate ou la zéolithe ZSM-4, et de préférence la zéolithe oméga, dont le diamètre des pores principaux est d'environ 7,4 Å et qui posséde un réseau microporeux monodimensionnel ("Atlas of Zeolites Structure Types", W.M. Meier et D.H. Olson, 3ème Edition, 1992).

La zéolithe de type structural mazzite, comprenant du silicium et au moins un élément T choisi dans le groupe formé par le gallium et l'aluminium, de préférence l'aluminium, a un rapport Si/T atomique global compris entre 3,2 et 100, de préférence entre 6 et 80, et de manière encore plus préférée entre 8 et 60, et a une teneur en sodium par rapport au poids de zéolithe sèche inférieure à 0,6 % poids, de préférence inférieure à 0,1 % poids

Dans le cas préféré où T est l'aluminium, on opère une désalumination d'une zéolithe de type structural mazzite brute de synthèse par toute méthode connue de l'homme du métier, en particulier selon le mode opératoire décrit dans le brevet US-A-4.780.436 lorsque T est l'aluminium, c'est-à-dire que l'on procède à une étape de calcination sous flux d'air sec, qui a pour but d'éliminer le structurant organique occlus dans la microporosité de la zéolithe, à au moins un échange ionique par au moins une solution de NH₄NO₃, de manière à éliminer pratiquement tout cation alcalin, en particulier le sodium, présent en position cationique dans la zéolithe, puis à au moins un cycle de désalumination de charpente, comportant au moins une calcination en présence de vapeur d'eau, à une température généralement comprise entre 550 et 850 °C, suivie d'au moins une attaque acide.

Dans le cas préféré où T est Al, le cycle de désalumination de la charpente, comportant au moins une étape de calcination sous vapeur d'eau et au moins une étape d'attaque en milieu acide de la zéolithe de type structural mazzite, peut être répété autant de fois qu'il est nécessaire pour obtenir la zéolithe de type structural mazzite désaluminée possédant les caractéristiques désirées. De même, suite au traitement de calcination sous vapeur d'eau, plusieurs attaques acides successives, avec des solutions en acide de concentration différentes, peuvent être opérées.

Le dépôt d'au moins un métal choisi dans l'ensemble formé par les métaux des groupes IIa, IVb, IIb et IVa, sur la surface extérieure des cristaux de ladite zéolithe, est réalisé généralement par greffage en utilisant comme agent de greffage au moins un composé organométallique dudit métal qui est généralement, d'une part, suffisamment volumineux pour ne pas pénétrer à l'intérieur du réseau microporeux de la zéolithe oméga et, d'autre part, susceptible de réagir avec les groupes OH de surface. Dans le cas de l'étain, on peut notamment employer comme agents de greffage, les composés de formule SnR¹R²R³R⁴ dans lesquels les groupes R¹, R², R³ et R⁴ identiques ou différents, sont des groupes organiques d'encombrement varié et, en général, d'encombrement important ; à titre d'exemples non limitatifs, on peut citer les radicaux alkyl, aryl, organosilyl vinyl, polynucléaire aryl, cyclo-alkyl, allyl, propargyl. Les groupes Rⁱ, i=1,2,3 ou 4, peuvent également être un groupe de type alkoxy ou aryl-oxy encombré, comme par exemple un groupe ter-butoxy, o,o'-diphényl-phénoxy, o,o'-di-isopropyl phénoxy etc... Le groupement Rⁱ peut aussi être un hydrure, mais il reste au moins un groupement Rⁱ organique fixé sur l'étain, tel que le cas des composés SnBu₂H₂ ou SnBu₃H.

Dans le cas général des métaux du groupe lla (Be, Mg, Ca, Sr, Ba, Ra) et des métaux des groupes IVb (Ti, Zr, Hf), IIb (Zn, Cd, Hg) et IVa (Ge, Sn, Pb), différents de l'étain, on peut utiliser, comme agents de greffage, également des composés de formule BeR₂, MgR₂, CaR₂, SrR₂, BaR₂, RaR₂, TiR₄, ZrR₄, HfR₄, CdR₂, HgR₂, GeR₄, SnR₄ ou PbR₄ dans lesquels Rⁱ, identiques ou différents entre eux, sont des groupes organiques tels que ceux définis précédemment.

Dans le cas de l'étain, un agent de greffage préféré est le tétrabutylétain noté SnBu₄. Dans le cas du magnésium, un agent de greffage préféré est le bis-néopentylmagnésium noté MgNp₂. Dans le cas du zirconium, un agent de greffage préféré est le tétranéopentylzirconium noté ZrNp₄. Dans le cas du germanium, un agent de greffage préféré est le tetrabutylgermanium noté GeBu₄.

La préparation de la zéolithe greffée comprend une première étape de prétraitement de la zéolithe de type structural mazzite, généralement par l'une des deux méthodes suivantes : une activation sous flux inerte, tel que l'azote, ou un prétraitement sous vide. Une deuxième étape est le greffage de la zéolithe de type structural mazzite. Ainsi, au moins un agent de greffage peut être fixé sur ladite zéolithe par une voie en phase gazeuse ou bien par une voie en phase liquide. Dans ce dernier cas, le composé organométallique choisi est alors placé en solution dans un solvant, par exemple l'hexane, sous gaz inerte. Une troisième étape consiste à décomposer les fragments organiques, ce qui se réalise généralement soit par la décomposition sous atmosphère oxydante, soit par décomposition sous vide.

Le dépôt éventuel d'au moins un autre métal choisi parmi les métaux des groupes IIa, IVb, IIb ou IVa, s'effectue de manière analogue selon les modes opératoires présentés précédemment.

A l'issue du traitement thermique de décomposition, la teneur pondérale de la zéolithe de type structural mazzite en métal (métaux) choisi(s) parmi l'un des groupes lla, lVb, IIb et/ou IVa est comprise entre 0.01 et 5% et, de manière avantageuse entre 0,01 et 4%.

Néanmoins, on peut éventuellement régler le niveau de sélectivité de la zéolithe de type structural mazzite en procédant au besoin à au moins un cycle supplémentaire "greffage de métal - calcination", suivant une des techniques décrites précédemment, de manière à atteindre une teneur pondérale en métal (métaux) supérieure à 5%, voire à 7% sur la zéolithe de type structural mazzite.

Les propriétés acides de la zéolithe de type structural mazzite ne sont pas altérées par le dépôt dudit (desdit) métal (métaux) selon les techniques décrites ci-dessus. Ainsi, la zéolithe de type structural mazzite obtenue possède une teneur pondérale en sodium par rapport au poids de zéolithe de type structural mazzite sèche inférieure à 2000 ppm, un rapport atomique Si/T global compris entre 5 et 100 et, de préférence entre 6 et 80 et de manière encore plus préférée entre 8 et 60.

Les caractéristiques de la zéolithe de type structural mazzite peuvent être mesurées par les méthodes suivantes:
- dans le cas préféré où T est l'aluminium, le rapport atomique Si/Al est déterminé par fluorescence X et par résonance magnétique nucléaire du silicium 29,
- la teneur en sodium est déterminé par absorption atomique,
- le volume de maille élémentaire et la cristallinité sont déterminés par diffraction X, l'échantillon de zéolithe oméga étant préparé comme dans le mode opératoire de la norme ASTM D3942 80 établie pour la faujasite.

La préparation du catalyseur être effectuée selon toute méthode connue de l'homme du métier. En général, elle est obtenue par mélange de la matrice et de la zéolithe puis par mise en forme. L'élément éventuel de l'ensemble formé par les groupe IB et VIII de la classification périodique des éléments peut être intoduit soit avant la mise en forme, ou bien lors du mélange, ou bien sur la zéolithe elle même avant de la mélanger, soit, de préférence, après la mise en forme. La mise en forme est généralement suivie d'une calcination, généralement à une température comprise entre 250 et 600 °C. L'élément éventuel de l'ensemble formé par les groupe IB et VIII de la classification périodique des éléments peut être intoduit après ladite calcination. Dans tous les cas, ledit élément est généralement déposé au choix soit, de préférence, pratiquement totalement sur la zéolithe, soit pratiquement totalement sur la matrice, soit en partie sur la zéolithe et en partie sur la matrice, ce choix s'effectuant, de la manière qui est connue de l'homme du métier, par les paramètres utilisés lors dudit dépôt, comme par exemple la nature du précurseur choisi pour effectuer ledit dépôt.

L'élément des groupes IB ou VIII, de préférence choisi dans le groupe formé par Ag, Ni et Pt, et de façon encore plus préférée Ni, peut également éventuellement être déposé sur le mélange zéolithe-matrice préalablement mis en forme par tout procédé connu de l'homme de l'art. Un tel dépôt est généralement effectué par la technique d'imprégnation à sec, d'échange(s) ionique(s) ou de coprécipitation. Dans le cas de l'échange ionique à partir de précurseurs à base d'argent, de nickel ou de platine, on utilise habituellement des sels d'argent tels que les chlorures ou les nitrates, un complexe tétramine du platine, ou des sels de nickel tels que les chlorures, les nitrates, les acétates ou les formiates. Cette technique d'échange cationique peut également être utilisée pour déposer directement le métal sur la poudre de zéolithe, avant son mélange éventuel avec une matrice.

Dans le cas où le catalyseur contient plusieurs métaux, ces derniers peuvent être introduits soit tous de la même façon soit par des techniques différentes, avant ou après mise en forme et dans n'importe quel ordre. Dans le cas où la technique utilisée est celle de l'échange ionique, plusieurs échanges successifs peuvent être nécessaires pour introduire les quantités requises de métaux.

Par exemple, une des méthodes préférées de préparation du catalyseur selon l'invention consiste à malaxer la zéolithe dans un gel humide de matrice (obtenu généralement par mélange d'au moins un acide et d'une poudre de matrice), par exemple d'alumine, pendant une durée nécéssaire pour l'obtention d'une bonne homogénéité de la pâte ainsi obtenue, soit par exemple pendant une dizaine de minutes, puis à passer ladite pâte à travers une filière pour former des extrudés, par exemple de diamètre compris entre 0,4 et 4 mm. Puis, après séchage pendant quelques minutes à 100 °C en étuve et après calcination, par exemple pendant 2 heures à 400 °C, l'élément éventuel, par exemple le nickel, peut être déposé, par exemple par échange ionique, ledit dépôt étant suivi d'une calcination finale, par exemple pendant 2 heures à 400 °C.

La mise en forme du catalyseur selon l'invention est généralement telle que le catalyseur est de préférence sous forme de pastilles, d'aggrégats, d'extrudés ou de billes, en vue de son utilisation.

La préparation du catalyseur se termine généralement par une calcination, dite calcination finale, habituellement à une température comprise entre 250 et 600 °C, de préférence précédée d'un séchage, par exemple à l'étuve, à une température généralement comprise entre la température ambiante et 250 °C, de préférence entre 40 et 200 °C. Ladite étape de séchage est de préférence menée pendant la montée en température nécessaire pour effectuer ladite calcination.

L'invention concerne l'utilisation dudit catalyseur en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation d'hydrocarbures alkylaromatiques généralement en C₉⁺ (c'est-à-dire à au moins 9 atomes de carbone par molécule), telle que la transalkylation et/ou la dismutation du toluène et/ou d'alkylaromatiques C₉⁺ pour produire des xylènes. La charge d'un tel procédé peut comprendre de 0 à 100 % d'alkylaromatiques en C₉⁺ et de 0 à 100 % de toluène.

Les conditions opératoires sont généralement les suivantes : une température comprise entre 250 et 600°C et de préférence entre 330 et 500°C ; une pression comprise entre 10 et 60 bar et de préférence entre 20 et 45 bar ; une vitesse spatiale d'alimentation, exprimée en kilogramme de charge introduite par kilogramme de catalyseur et par heure, comprise entre 0,1 et 10 et de préférence entre 0,5 et 4 ; un rapport molaire hydrogène sur hydrocarbures compris entre 2 et 20 et de préférence entre 3 et 12.

Les exemples suivants illustrent l'invention sans toutefois en limiter la portée.

### Exemple 1 : Préparation des zéolithes 1 et 2 selon l'invention

La matière première utilisée est une zéolithe oméga, qui possède un rapport atomique Si/Al global égal à 3,2, une teneur pondérale en sodium par rapport au poids en zéolithe oméga sèche d'environ 5,3 %, un volume de maille élémentaire de 2,196 nm³, et un volume poreux, à l'azote, mesuré à -196°C et à P/Pₒ=0,19 de 0,125 cm³ liquide par gramme.

Cette zéolithe oméga subit tout d'abord une calcination dite sèche à 550°C sous flux d'air et d'azote durant 6 heures. Puis, le solide obtenu est soumis à trois échanges ioniques dans une solution de NH₄NO₃ 10N, à environ 100°C pendant 4 heures pour chaque échange. La zéolithe oméga est alors soumise à un traitement hydrothermique, en présence de 50% de vapeur d'eau à 600°C, durant 4 heures. La zéolithe subit alors une attaque acide, à l'aide d'une solution d'acide nitrique 1N, à environ 100°C, pendant 2 heures, de manière à extraire les espèces aluminiques extra-réseau formées lors du traitement hydrothermique. Le volume V de la solution d'acide nitrique engagé (en ml) est égal à 10 fois le poids P de zéolithe oméga sèche (V/P=10). Puis la zéolithe oméga précédemment traitée est soumise de nouveau à un traitement hydrothermique, en présence de 50% de vapeur d'eau mais cette fois ci à 700°C, durant 4 heures, puis à une attaque acide, par une solution d'acide nitrique 1.5N à environ 100°C, pendant 4 heures, de manière à extraire les espèces aluminiques extra-réseau formées lors du traitement hydrothermique. Le volume V de la solution d'acide nitrique engagé (en ml) est égal à 10 fois le poids P de zéolithe oméga sèche (V/P=10).

A l'issue de ces traitements, la zéolithe oméga sous forme H a un rapport Si/AI atomique global égal à environ 25, une teneur pondérale en sodium par rapport au poids de zéolithe oméga sèche de 90 ppm, un volume de maille élémentaire de 2,115 nm³, et une capacité d'adsorption en azote mesurée à -196°C et à P/Pₒ=0,19 de 0,206 cm³ N2 liquide/g.

L'étain est ensuite greffé sur la surface externe des cristaux de la zéolithe oméga forme H par la voie en phase liquide décrite précédemment. La décomposition des fragments organiques liés à l'étain après l'étape de greffage peut se faire sous vide ou bien en présence d'air (atmosphère oxydante).

### i) Méthode de greffage en phase liquide et décomposition des fragments organiques sous vide.

On opère selon les étapes successives suivantes :
- Activation de la zéolithe oméga, par traitement sous gaz inerte à 150°C durant 12 heures, puis diminution de la température à 20°C, sous inerte.
- Calcination des éventuelles traces de composés organiques adsorbés dans la zéolithe sous flux d'air sec (0,4 l/h/g) et de gaz inerte (2 l/h/g) durant 2 heures à 550°C, puis uniquement sous flux d'air sec (2 l/h/g) durant 2 heures toujours à 550°C.
- La zéolithe oméga est refroidie à 20°C sous gaz inerte et mise en suspension dans l'hexane. Le tétrabutylétain est alors injecté, toujours sous atmosphère de gaz inerte.
- Après 15 minutes d'agitation, le solvant est éliminé sous vide. La température de la zéolithe oméga est alors augmentée jusqu'à 150°C, puis maintenue durant 6 heures, sous atmosphère inerte. Après refroidissement à tempérautre ambiante de la zéolithe, l'excès de SnBu₄ est éliminé par rinçage avec une solution d'hexane frais.
- Les fragments organiques butyles liés à l'étain sont alors décomposés par traitement thermique sous vide. Pour cela, la zéolithe oméga est placée sous vide dynamique durant 2 heures à la température de 450°C, puis sous flux de gaz inerte durant 15 heures.

Le solide obtenu à l'issue de ces traitements est référencé Ω1 : sa teneur pondérale en étain est de 1,3%. Ses autres caractéristiques restent inchangées par rapport à celles de la zéolithe oméga forme H.

### ii) Méthode de greffage en phase liquide et décomposition des fragments organiques sous atmosphère oxydante

Le mode d'obtention de la zéolithe oméga et son mode de greffage sont identiques à ceux décrits précédemment conduisant à la préparation du solide référencé Ω1. Seul change le mode de décomposition des fragments organiques butyles liés à l'étain qui se fait sous atmosphère oxydante, qui est réalisé en présence d'un mélange d'oxygène et de gaz inerte, à 450°C durant 4 heures.

Le solide ainsi obtenu est référencé Ω2 : sa teneur pondérale en étain est de 1,26 %. Ses autres caractéristiques restent inchangées par rapport à celles de la zéolithe oméga forme H.

### Exemple 2 : Préparation des catalyseurs C3 et C4 conformes à l'invention

La zéolithe Ω1, greffée, obtenue à l'exemple 1 est ensuite mise en forme par extrusion avec un gel d'alumine de manière à obtenir, après séchage et calcination sous air sec, le catalyseur C3 qui contient en % poids 80 % de zéolithe oméga greffée et 20 % d'alumine.

De même la zéolithe Ω2 obtenue à l'exemple 1 est ensuite mise en forme par extrusion avec un gel d'alumine de manière à obtenir, après séchage et calcination sous air sec, le catalyseur C4 qui contient en % poids 80 % de zéolithe oméga greffée et 20 % d'alumine.

### Exemple 3 : Préparation du catalyseur C5 conforme à l'invention

Dans cet exemple, le catalyseur C4 de l'exemple 2 est soumis à 3 échanges ioniques avec une solution d'acétate de nickel de manière à introduire 1 % poids de nickel dans le catalyseur.

Pour cela, le catalyseur C4 est mis en contact avec une solution 0,5 M de Ni(CH₃CO₂)₂ à température ambiante et sous agitation. Entre chaque échange, le solide est séparé de la solution d'imprégnation et lavé abondamment à l'eau permutée. La concentration de la solution d'imprégnation est pour chaque échange ré-ajustée à la concentration de 0,5 mole par litre.

Le solide humide est ensuite séché à 120°C pendant 12 heures, et calciné sous un débit d'air sec à la température de 500°C pendant 1 heure. Le catalyseur C5 ainsi obtenu contient, en % poids, 79,32 % de zéolithe oméga greffée, forme hydrogène, 19,83 % d'alumine et 0,85 % de Nickel.

### Exemple 5 : Préparation du catalyseur C6 conforme à l'invention

La zéolithe oméga utilisée pour préparer le catalyseur C6 est la zéolithe désaluminée sous forme hydrogène préparée dans l'exemple 1 et qui posséde les caractéristiques suivantes rapport Si/Al atomique global égal à environ 25, une teneur pondérale en sodium par rapport au poids de zéolithe oméga sèche de 90 ppm, un volume de maille élémentaire de 2,115 nm³, et une capacité d'adsorption en azote mesurée à -196°C et à P/Pₒ=0,19 de 0,206 cm³ N2 liquide/g.

On dépose ensuite le germanium sur la surface externe des cristaux de ladite zéolithe oméga suivant la méthode en phase gazeuse. On opère selon les étapes successives suivantes :
- Activation de la zéolithe sous vide dynamique, à la température de 160°C, durant 8 heures. La température est alors augmentée à la vitesse de 2°C/min jusqu'à 450°C, et maintenue durant 9 heures sous vide dynamique. La température est alors abaissée jusqu'à 250°C, température à laquelle le GeBu₄ est injecté. La zéolithe oméga est alors laissée sous tension de vapeur de GeBu₄ durant 48 heures à 250°C. Après retour à la température ambiante (20°C), les gaz dégagés sont éliminés sous vide dynamique, puis les espèces physisorbées résiduelles sont désorbées sous vide dynamique durant 8 heures à 220°C. Les fragments butyles fixés sur le germanium sont ensuite décomposés par traitement thermique sous atmosphère oxydante. Ce traitement est réalisé en présence d'un mélange d'oxygène et de gaz inerte, à 450°C durant 4 heures.

Le solide obtenu à l'issue de ces traitements est référencé Ω6 : sa teneur pondérale en germanium est de 0,31%. Ses autres caractéristiques restent inchangées par rapport à celles de la zéolithe oméga forme H.

La zéolithe oméga greffée, Ω6, précédemment obtenue est ensuite mise en forme par extrusion avec un gel d'alumine de manière à obtenir, après séchage et calcination sous air sec, un solide extrudé qui contient en % poids 80 % de zéolithe oméga et 20 % d'alumine.

Les extrudés obtenus sont mis en contact avec une solution 0,5 M de Ni(CH₃CO₂)₂ à température ambiante et sous agitation. Entre chaque échange, le solide est séparé de la solution d'imprégnation et lavé abondamment à l'eau permutée. La concentration de la solution d'imprégnation est pour chaque échange ré-ajustée à la concentration de 0,5 mole par litre.

Le solide humide est ensuite séché à 120°C pendant 12 heures, et calciné sous un débit d'air sec à la température de 500°C pendant 1 heure. Le catalyseur C6 ainsi obtenu contient, en % poids, 79,32 % de zéolithe oméga forme hydrogène, 19,83 % d'alumine et 0,85 % de nickel.

### Exemple 6 : Préparation du catalyseur C7 non conforme à l'invention

La matière première utilisée est la même zéolithe oméga sous forme H que celle préparée dans l'exemple 1. Elle possède un rapport Si/Al atomique global égal à environ 25, une teneur pondérale en sodium par rapport au poids de zéolithe oméga sèche de 90 ppm, un volume de maille élémentaire de 2,115 nm³, et une capacité d'adsorption en azote mesurée à -196°C et à P/Pₒ=0,19 de 0,206 cm³ N2 liquide/g.

Cette zéolithe ne subit pas d'étape de greffage par au moins un élément choisi parmi les métaux du groupe IIa, IVb, IIb et IVa.

La zéolithe oméga non greffée est ensuite mise en forme par extrusion avec un gel d'alumine de manière à obtenir, après séchage et calcination sous air sec, un solide extrudé qui contient en % poids 80 % de zéolithe oméga et 20 % d'alumine.

Les extrudés obtenus sont mis en contact avec une solution 0,5 M de Ni(CH₃CO₂)₂ à température ambiante et sous agitation. Entre chaque échange, le solide est séparé de la solution d'imprégnation et lavé abondamment à l'eau permutée. La concentration de la solution d'imprégnation est pour chaque échange ré-ajustée à la concentration de 0,5 mole par litre.

Le solide humide est ensuite séché à 120°C pendant 12 heures, et calciné sous un débit d'air sec à la température de 500°C pendant 1 heure. Le catalyseur C7 ainsi obtenu contient, en %poids, 79,32 % de zéolithe oméga forme hydrogène exempte d'espèces aluminiques extra-réseau, 19,83 % d'alumine et 0,85 % de Nickel.

Un synopsis des compositions des catalyseurs C3, C4, C5, C6 et C7 est donné dans le tableau suivant (en % poids) :

| Catalyseurs | Teneur Sn ou Ge par rapport à la zéolithe | Nickel par rapport au catalyseur | Alumine | Teneur zéolithe grefée dans le catalyseur |
|---|---|---|---|---|
| **C3** | 1,3 (Sn) | 0 | 20 | 80 |
| **C4** | 1,26 (Sn) | 0 | 20 | 80 |
| **C5** | 1,26 (Sn) | 0,85 | 19,83 | 79,32 |
| **C6** | 0,31 (Ge) | 0,85 | 19,83 | 79,32 |
| **C7** | 0 | 0,85 | 19,83 | 79,32 |

### Exemple 7 : Evaluation des performances des catalyseurs

Les catalyseurs sont mis en oeuvre dans un réacteur à lit fixe, sous pression, dans lequel est introduit la charge qui est constituée de toluène pur.

La comparaison des rendements en (benzène + ethylbenzène + xylènes) obtenus en utilisant les catalyseurs C5 et C6, conformes à l'invention, et C7, non conforme à l'invention, est effectuée dans le tableau ci-après:

| Catalyseurs | C5 (conforme) | C6 (conforme) | C7 (non conforme) |
|---|---|---|---|
| Température de réaction (°C) | 430 | 430 | 430 |
| Pression totale de réaction (Bar) | 40 | 40 | 40 |
| Rendements % poids (Benzène + Ethylbenzène + xylènes) | 37,8 | 37,6 | 36,9 |

La comparaison des catalyseurs C5 et C6 et d'autre part la comparaison du catalyseur C7, montre que les catalyseurs selon l'invention, C5 et C6, conduisent à des rendements en (Benzène + Ethylbenzène + xylènes) supérieurs à ceux obtenus avec le catalyseur non conformes C7.

## Revendications

1. Utilisation en dismutation d'hydrocarbures alkylaromatiques et/ou en transalkylation d'hydrocarbures alkylaromatiques d'un catalyseur comportant au moins une zéolithe de type structural mazzite, telle qu'elle comprend, sur la surface extérieure de ses cristaux, au moins un élément choisi parmi les métaux des groupes IIa, IVb, IIb et IVa de la classification périodique des éléments, ledit catalyseur comprenant aussi au moins une matrice et éventuellement au moins un élément choisi dans l'ensemble formé par les groupes IB et VIII de la classification périodique des éléments.

2. Utilisation selon la revendication 1 en transalkylation et/ou dismutation de toluène et/ou d'alkylaromatiques à au moins 9 atomes de carbone par molécule.

3. Utilisation selon l'une des revendications 1 ou 2 telle que ladite zéolithe est choisie dans le groupe formé par la zéolithe mazzite gallosilicate, la mazzite, la zéolithe LZ-202, la zéolithe oméga ou la zéolithe ZSM-4.

4. Utilisation selon l'une des revendications 1 à 3 telle que ladite zéolithe est une zéolithe oméga.

5. Utilisation selon l'une des revendications 1 à 4 telle que ledit catalyseur contient de 10 à 99% de zéolithe de type stuctural mazzite et éventuellement, dans le cas où le catalyseur contient au moins un élément choisi dans l'ensemble formé par les groupes IB et VIII de la classification périodique des éléments, la teneur pondérale du(des)dit(s) élément(s) est comprise entre 0,01% et 10%, le complément à 100 % poids consistant en la part de matrice du catalyseur.

6. Utilisation selon l'une des revendications 1 à 5 telle que la matrice est choisie parmi les éléments du groupe formé par les argiles, la magnésie, les alumines, les silices, l'oxyde de titane, l'oxyde de bore, la zircone, les phosphates d'aluminium, les phosphates de titane, les phosphates de zirconium et les silice-alumines.

7. Utilisation selon l'une des revendications 1 à 6 telle que le dépôt d'au moins un métal choisi dans l'ensemble formé par les métaux des groupes IIa, IVb, IIb et IVa est réalisé par greffage en utilisant comme agent de greffage au moins un composé organométallique dudit métal.

8. Utilisation selon l'une des revendications 1 à 7 opérée selon les conditions suivantes : une température comprise entre 250 et 600°C, une pression comprise entre 10 et 60 bar, une vitesse spatiale d'alimentation, exprimée en kilogramme de charge introduite par kilogramme de catalyseur et par heure, comprise entre 0,1 et 10, et un rapport molaire hydrogène sur hydrocarbures compris entre 2 et 20.

## Patentansprüche

1. Verwendung eines Katalysators zur Dismutation und/oder zur Transalkylierung von alkylaromatischen Kohlenwasserstoffen, die wenigstens einen Zeolith mit Mazzitstruktur aufweisen, derart, dass dieser auf der äußeren Oberfläche seiner Kristalle wenigstens ein Element umfasst, das aus den Metallen der Gruppen IIa, IVb, IIb und IVa des Periodensystems der Elemente gewählt wurde, und wobei dieser Katalysator außerdem mindestens eine Matrix und gegebenenfalls mindestens ein Element gewählt aus der Gesamtmenge der Gruppen IB und VIII des Periodensystems der Elemente umfasst.

2. Verwendung nach Anspruch 1 zur Transalkylierung und/oder Dismutation von Toluol und/oder Alkylaromaten mit wenigstens 9 Kohlenstoffatomen pro Molekül.

3. Verwendung nach einem der Ansprüche 1 oder 2, derart, dass der Zeolith aus der durch die Zeolith-Mazzit-Gallosilikate, Mazzit, Zeolith LZ-202, Omega-Zeolith oder Zeolith ZSM-4 gebildete Gruppe gewählt wurde.

4. Verwendung nach einem der Ansprüche 1 bis 3, derart, dass der Zeolith ein Omega-Zeolith ist.

5. Verwendung nach einem der Ansprüche 1 bis 4, derart, dass der Katalysator 10 bis 99% des Zeolithes mit Mazzitstruktur enthält und gegebenenfalls, in dem Fall, dass der Katalysator mindestens ein Element, gewählt aus der Gesamtmenge der Gruppen IB und VIII des Periodensystems der Elemente, enthält, liegt der Gewichtsanteil dieser Elemente zwischen 0,01% und 10% und der Rest besteht bis auf 100 Gew.-% aus dem Matrix-Teil des Katalysators.

6. Verwendung nach einem der Ansprüche 1 bis 5, derart, dass die Matrix gewählt ist aus den Elementen der Gruppe, die aus Tonen, Magnesiumoxid, Aluminiumoxiden, Siliziumoxiden, Titanoxid, Boroxid, Zirkonoxid, Aluminiumphosphaten, Titanphosphaten, Zirkonoxidphosphaten und Siliziumaluminiumoxiden gebildet wird.

7. Verwendung nach einem der Ansprüche 1 bis 6, derart, dass die Abscheidung mindestens eines Metalls, das aus der Gesamtmenge gewählt ist, die durch die Gruppen IIa, IVb, IIb und IVa gebildet wird, mittels Pfropfen realisiert ist, indem als Pfropfenmittel wenigstens eine organometallische Verbindung dieses Metalls verwendet wird.

8. Verwendung nach einem der Ansprüche 1 bis 7 betrieben unter den folgenden Bedingungen: eine Temperatur zwischen 250 und 600°C, ein Druck zwischen 10 und 60 bar, eine Volumengeschwindigkeit des Nahrungsmittels zwischen 0,1 und 10, ausgedrückt in Kilogramm zugeführte Charge pro Kilogramm Katalysator pro Stunde, und ein Molverhältnis Wasserstoff zu Kohlenwasserstoffen zwischen 2 und 20.

## Claims

1. The use of a catalyst comprising at least one zeolite with structure type mazzite, the catalyst comprising, on the external surface of its crystals, at least one metal selected from metals from groups IIa IVb, IIb and IVa of the periodic classification of the elements, said catalyst also comprising at least one matrix and, optionally at least one element selected from the group formed by groups IB and VIII of the periodic classification of the elements, for the dismutation of alkylaromatic hydrocarbons and/or for the transalkylation of alkylaromatic hydrocarbons.

2. Use according to claim 1, for the transalkylation and/or dismutation of toluene and/or alkylaromatics containing at least 9 carbon atoms per molecule.

3. Use according to claim 1 or claim 2, in which said zeolite is selected from the group formed by gallosilicate mazzite zeolite, mazzite, LZ-202 zeolite, omega zeolite, and ZSM-4 zeolite.

4. A catalyst according to any one of claims 1 to 3, in which said zeolite is omega zeolite.

5. Use according to any one of claims 1 to 4, in which said catalyst contains 10% to 99% of zeolite with structure type mazzite and, optionally, when the catalyst contains at least one element selected from the group formed by groups IB and VIII of the periodic classification of the elements, said element(s) are present in an amount which is in the range 0.01% to 10% by weight, the complement to 100% by weight consisting of the matrix of the catalyst.

6. Use according to any one of claims 1 to 5, in which the matrix is selected from members of the group formed by clays, magnesia, aluminas, silicas, titanium oxide, boron oxide, zirconia, aluminium phosphates, titanium phosphates, zirconium phosphates and silica-aluminas.

7. Use according to any one of claims 1 to 6, in which deposition of at least one metal selected from the group formed by metals from groups IIa, IVb, IIb and IVa is carried out by grafting using at least one organometallic compound of said metal as a grafting agent.

8. Use according to any one of claims 1 to 7, carried out under the following conditions: a temperature which is in the range 250°C to 600°C; a pressure which is in the range 10 to 60 bar; a supply space velocity, expressed in kilograms of feed introduced per kilogram of catalyst per hour, in the range 0.1 to 10; and a hydrogen to hydrocarbons molar ratio which is in the range 2 to 20.
